(19) Europäisches Patentamt / European Patent Office / Office européen des brevets

(11) **EP 4 740 930 A1**

(12) **DEMANDE DE BREVET EUROPEEN**

(43) Date de publication:
**13.05.2026 Bulletin 2026/20**

(21) Numéro de dépôt: **25212939.0**

(22) Date de dépôt: **03.11.2025**

(51) Classification Internationale des Brevets (IPC):
*A61K 8/06* $^{(2006.01)}$    *A61K 8/34* $^{(2006.01)}$
*A61K 8/37* $^{(2006.01)}$    *A61K 8/49* $^{(2006.01)}$
*A61Q 1/00* $^{(2006.01)}$    *A61Q 19/00* $^{(2006.01)}$
*A61Q 19/10* $^{(2006.01)}$

(52) Classification Coopérative des Brevets (CPC):
**A61K 8/068; A61K 8/062; A61K 8/345;
A61K 8/375; A61K 8/4993; A61Q 1/00;
A61Q 19/00; A61Q 19/10**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **06.11.2024 FR 2412132**

(71) Demandeur: **Dermosciences France**
**06800 Cagnes-sur-Mer (FR)**

(72) Inventeurs:
• **POT, Monory**
**06800 CAGNES-SUR-MER (FR)**
• **ACHILLE, Marie-Anne**
**06800 CAGNES-SUR-MER (FR)**

(74) Mandataire: **Bandpay & Greuter**
**11, rue Christophe Colomb**
**75008 Paris (FR)**

(54) **MICROÉMULSION HUILE-DANS-EAU STABLE**

(57) La présente invention concerne une composition sous forme d'une microémulsion huile-dans-eau ayant une excellente stabilité au cours du temps. En particulier, la composition selon l'invention comprend un triglycéride d'acide gras à chaîne moyenne, un tensioactif à faible HLB, un tensioactif à haut HLB, un diol choisi parmi le 1,3-propanediol et le propylène glycol et de l'eau. L'invention concerne également l'utilisation cosmétique de ladite composition et un procédé d'application de ladite composition sur les matières kératiniques.

EP 4 740 930 A1

## Description

### Domaine technique

La présente invention concerne une composition sous forme d'une microémulsion huile-dans-eau ayant une excellente stabilité au cours du temps. En particulier, la composition selon l'invention comprend un triglycéride d'acide gras à chaîne moyenne, un tensioactif à faible HLB, un tensioactif à haut HLB, un diol et de l'eau. L'invention concerne également l'utilisation cosmétique de ladite composition et un procédé d'application de ladite composition sur les matières kératiniques.

### État de la technique antérieure

Les préparations cosmétiques et dermatologiques destinées aux soins de la peau et des phanères contiennent généralement des ingrédients solubles dans l'huile et des ingrédients solubles dans l'eau, souvent sous forme d'émulsions. Une émulsion est un mélange de deux ou plusieurs liquides non miscibles ou partiellement miscibles, où un liquide, appelé phase dispersée ou phase discontinue, est dispersé en petites gouttelettes dans l'autre, appelé phase continue. Les émulsions sont classées en fonction de leur phase continue, en émulsions huile-dans-eau (H/E) ou eau-dans-huile (E/H).

Les microémulsions représentent une catégorie spécifique d'émulsions, obtenues à partir de quatre composants : une phase aqueuse, une phase huileuse, un tensioactif et un co-tensioactif. La composition obtenue conduit à une microstructure formée de microgouttelettes dont la taille est inférieure à 100 nanomètres. Cette taille de gouttelettes réduite permet à la lumière de traverser la microémulsion sans être diffusée, lui conférant un aspect transparent, contrairement à l'apparence opaque des émulsions classiques.

Cette propriété confère aux microémulsions des avantages significatifs dans les formulations cosmétiques. Elles offrent une meilleure pénétration des actifs grâce à la petite taille des gouttelettes, une transparence optique due à l'absence de diffusion de la lumière, et la possibilité d'être préparées à froid. Cette dernière caractéristique permet de réduire la consommation d'énergie lors de la fabrication et d'utiliser des actifs thermosensibles qui pourraient être dégradés dans une émulsion classique. Un autre avantage des microémulsions réside dans leur capacité à contenir une plus grande quantité d'actifs difficiles à solubiliser.

Malgré ces avantages, la stabilité à long terme des microémulsions demeure un défi technique majeur. Bien que généralement considérées comme thermodynamiquement stables, les microémulsions peuvent être sujettes à des phénomènes de déstabilisation au cours du stockage, tels que la sédimentation ou la coalescence. Ces processus peuvent altérer les propriétés physico-chimiques et l'efficacité du produit cosmétique avec le temps. Bien que l'augmentation de la concentration en tensioactif puisse améliorer la stabilité des microémulsions, des concentrations élevées peuvent résulter en une texture grossière, collante ou grasse, altérant ainsi les propriétés organoleptiques du produit.

A. Shah et al décrit la formation de microémulsions à partir de triglycérides à chaîne moyenne comme phase lipophile et de polysorbates comme tensioactifs (A. Shah et al, Drug Development and Industrial Pharmacy, 2017, vol. 44, pages 215-223). Ces microémulsions ne se forment qu'à des ratios très bas de phase lipophile/tensioactif (1:9 ou 2:8), nécessitant ainsi des quantités très élevées de tensioactifs. Le document décrit également des microémulsions préparées à partir d'un polysorbate, un triglycéride à chaîne moyenne et un monoglycéride. En reproduisant l'enseignement de ce document, la Demanderesse a observé que ces microémulsions tendent à perdre leur stabilité au cours du temps.

Le document US2011/159104 décrit des microémulsions à base de miel et de produits apicoles comprenant des triglycérides, des tensioactifs à faible et à haut HLB ainsi que des co-solvants et de l'eau.

Il existe donc un besoin de développer une composition sous forme de microémulsion, notamment pour des applications cosmétiques et/ou dermatologiques, qui combine les avantages des microémulsions en termes d'efficacité et de propriétés organoleptiques, tout en offrant une stabilité améliorée capable de résister aux conditions de stockage à long terme.

### Résumé de l'invention

Selon un premier aspect, l'invention concerne une composition sous forme de microémulsion huile-dans-eau comprenant au moins :

> a) un triglycéride d'acide gras à chaîne moyenne ;
> b) un tensioactif à faible HLB ;
> c) un tensioactif à haut HLB ;
> d) un diol choisi parmi le 1,3-propanediol et le propylène glycol;
> e) de l'eau.

Avantageusement, le tensioactif à faible HLB a un HLB allant de 3 à 6, de préférence de 5 à 6 et/ou le tensioactif à haut HLB a un HLB allant de 13 à 20, de préférence de 14 à 18.

Avantageusement, le rapport massique tensioactif à haut HLB au mélange (triglycéride d'acide gras à chaîne moyenne + tensioactif à faible HLB) est supérieur ou égal à 0,85, de préférence va de 0,90 à 5, de préférence encore de 0,90 à 2.

Selon un aspect particulier, l'invention concerne une composition sous forme de microémulsion huile-dans-eau comprenant au moins :

a) un triglycéride d'acide gras à chaîne moyenne ayant de 6 à 12 atomes de carbone;
b) un tensioactif à faible HLB ayant un HLB allant de 3 à 6 ;
c) un tensioactif à haut HLB ayant un HLB allant de 13 à 20;
d) un diol choisi parmi le 1,3-propanediol et le propylène glycol ;
e) de l'eau,

dans laquelle le rapport massique du tensioactif à haut HLB au mélange (triglycéride d'acide gras à chaîne moyenne ayant de 6 à 12 atomes de carbone + tensioactif à faible HLB) est supérieur ou égal à 0,85.

Avantageusement, la composition selon l'invention comprend de 3% à 20% en poids, de préférence de 3% à 15% en poids, de préférence encore de 4% à 12% en poids, de préférence encore de 5 à 10% en poids de diol par rapport au poids total de la composition.

Selon un mode de réalisation, le diol est le 1,3-propanediol.

Avantageusement, le triglycéride d'acide gras à chaîne moyenne est le triglycéride caprylique/caprique.

Avantageusement, le tensioactif à faible HLB comprend un monoglycéride et/ou un diglycéride d'acide gras à chaîne moyenne, de préférence choisi parmi le glycéryl monocaprate, glycéryl dicaprate, glycéryl monocaprylate, glycéryl dicaprylate, glycéryl caprate, glycéryl caprylate, glycéryl monocaprylate/caprate, glycéryl caprylate/caprate, glycéryl dicaprylate/caprate, de préférence encore est le glycéryl caprylate.

Selon un mode de réalisation, le tensioactif à haut HLB comprend un ester de sorbitane éthoxylé, de préférence le polysorbate 80.

Avantageusement, la composition selon l'invention comprend, par rapport au poids total de la composition :

- de 7% à 9% en poids de triglycéride d'acide gras à chaîne moyenne ;
- de 12% à 15% en poids de tensioactif à faible HLB;
- de 20% à 24% en poids de tensioactif à haut HLB ;
- de 5% à 10% en poids de diol ; et
- de 42% à 56% en poids d'eau.

Avantageusement, la composition selon l'invention comprend en outre au moins un agent cosmétiquement et/ou dermatologiquement acceptable et/ou au moins un actif biologique.

L'invention concerne également l'utilisation d'une composition selon l'invention comme produit de maquillage, d'hygiène, de nettoyage et/ou de soin des matières kératiniques, de préférence de la peau.

L'invention concerne également l'utilisation cosmétique non thérapeutique d'une composition selon l'invention pour prévenir et/ou traiter les signes du vieillissement cutané, de préférence pour réduire et/ou éliminer et/ou atténuer les rides et les ridules.

L'invention concerne également l'utilisation cosmétique non thérapeutique d'une composition selon l'invention pour prévenir et/ou réduire et/ou éliminer l'acné et/ou pour favoriser l'élimination des effets cosmétiques de l'acné ou les taches d'acné.

L'invention concerne également l'utilisation cosmétique non thérapeutique d'une composition selon l'invention pour prévenir et/ou réduire et/ou atténuer et/ou éliminer les taches de pigmentation, et/ou réduire leur taille, et/ou unifier et/ou illuminer et/ou éclaircir le teint.

La Demandeuse a mis au point des compositions sous forme de microémulsion huile-dans-eau stables, en optimisant le choix des ingrédients et notamment leurs proportions. Les microémulsions selon l'invention ont montré une stabilité exceptionnelle sur plusieurs mois, voire plusieurs années, même lorsqu'elles sont soumises à des températures de stockage élevées.

**Description détaillée**

L'expression « consiste essentiellement en » suivie d'une ou plusieurs caractéristiques, signifie que peuvent être inclus dans le procédé ou le matériau de l'invention, outre les composants ou étapes explicitement énumérés, des composants ou des étapes qui ne modifient pas significativement les propriétés et caractéristiques de l'invention.

L'expression « compris entre X et Y » inclut les bornes, sauf mention contraire explicite. Cette expression signifie donc que l'intervalle visé comprend les valeurs X, Y et toutes les valeurs allant de X à Y.

Les différents modes de réalisation, variantes, les préférences et les avantages décrits pour chacun des objets de l'invention s'appliquent à tous les objets de l'invention et peuvent être pris séparément ou en combinaison.

Par « microémulsion », on entend au sens de la présente invention un système colloïdal transparent ou translucide constitué de deux phases non miscibles, notamment une phase lipophile et une phase hydrophile, stabilisés par un tensioactif ou un mélange d'un tensioactif et d'un co-tensioactif. Contrairement aux émulsions classiques, qui peuvent être opalescentes ou turbidées, les microémulsions sont limpides et homogènes avec un diamètre des domaines dispersés ou gouttelettes variant de 1 à 100 nm.

Dans la suite de la description et des revendications, le terme « microémulsion » désigne exclusivement une émulsion huile-dans-eau. Cela signifie que les gouttelettes de la phase lipophile sont dispersées dans la phase hydrophile aqueuse, qui constitue la phase continue.

Par « lipophile » on entend au sens de l'invention une phase et/ou un composé qui a une affinité ou une tendance à se mélanger avec les graisses, les huiles et/ou les solvants non polaires.

Par « hydrophile » on entend au sens de l'invention une phase et/ou un composé qui a une affinité ou une tendance à se mélanger avec l'eau ou les solutions aqueuses. Dans le contexte de l'invention, la phase hydrophile aqueuse est la phase continue de la microémulsion.

Par « tensioactif » ou « émulsifiant » ou « surfactant », on entend, au sens de l'invention, un composé possédant une structure amphiphile capable de réduire la tension superficielle entre deux phases non miscibles, notamment une phase lipophile et une phase hydrophile, permettant ainsi la formation et la stabilisation de gouttelettes de l'une des phases dispersées dans l'autre.

Le terme « HLB » aussi appelé « Hydrophilic-Lipophilic Balance» en anglais ou encore "équilibre hydrophile-lipophile", désigne la valeur qui caractérise l'équilibre existant entre la partie hydrophile et la partie lipophile d'une molécule de tensioactif. La valeur HLB est généralement déterminée en calculant les pourcentages des poids moléculaires des parties hydrophiles et

$$HLB = 20 * M_h/M$$

lipophiles de la molécule de tensioactif selon la formule suivante : dans laquelle $M_h$ est la masse moléculaire de la portion hydrophile de la molécule, et M est la masse moléculaire de la molécule entière, donnant un résultat sur une échelle de 0 à 20. Une valeur HLB de 0 correspond à une molécule complètement lipophile, et une valeur de 20 correspond à une molécule complètement hydrophile. Une telle méthode de détermination du HLB est décrite par exemple dans Griffin, William C, Calculation of HLB Values of Non-Ionic Surfactants, Journal of the Society of Cosmetic Chemists, 1954, 5 (4): 249-56.

Composition selon l'invention

Selon un premier aspect, l'invention concerne une composition sous forme de microémulsion huile-dans-eau comprenant au moins :

a) un triglycéride d'acide gras à chaîne moyenne ;
b) un tensioactif à faible HLB,
c) un tensioactif à haut HLB ;
d) un diol choisi parmi le 1,3-propanediol et le propylène glycol ; et
e) de l'eau.

Les triglycérides d'acide gras à chaîne moyenne utilisés dans la composition selon la présente invention sont des esters dérivés de la combinaison de glycérol et d'acides gras à chaîne moyenne. Ils peuvent être d'origine naturelle, semi-synthétique ou synthétique et peuvent inclure des mélanges de différents triglycérides d'acides gras à chaîne moyenne.

Par "acide gras à chaîne moyenne", on entend au sens de l'invention un acide gras comportant de 6 à 12 atomes de carbone, tel que l'acide caproïque (acide hexanoïque, C6), l'acide caprylique (acide octanoïque, C8), l'acide caprique (acide décanoïque, C10) et l'acide laurique (acide dodécanoïque, C12).

Au sens de l'invention, l'expression "triglycéride d'acides gras à chaîne moyenne" désigne donc un triglycéride constitué d'acides gras comportant de 6 à 12 atomes de carbone.

De préférence, les triglycérides d'acide gras à chaîne moyenne sont des triglycérides d'acide caprylique (octanoïque, C8) et d'acide caprique (décanoïque, C10), notamment appelés triglycérides caprylique/caprique.

Ces triglycérides sont généralement obtenus à partir d'huiles végétales telles que l'huile de noix de coco et l'huile de palmiste. Les triglycérides caprylique/caprique peuvent comprendre de 50 à 100% en poids d'acide caprylique et de 0 à 50% en poids de triglycérides d'acide caprique.

Des exemples de triglycérides caprylique/caprique incluent ceux disponibles sous les noms commerciaux MYRITOL (Ami Ingredients); CAPTEX (Karlshams Lipid Specialties), par exemple CAPTEX 355, CAPTEX 300, CAPTEX 350, CAPTEX 850 et CAPTEX 8000; DUB MCT 5545 (Stearinerie Dubois) ; Crodamol™ GTCC (Croda); Labrafac CC MB (Gattefossé) ; SABODERM TCC MB (SABO S.p.A).

Avantageusement, la composition sous forme de microémulsion huile-dans-eau selon l'invention comprend de 3 à 15% en poids, de préférence de 5 à 12% en poids, de préférence encore de 7 à 9% de triglycérides d'acide gras à chaîne moyenne par rapport au poids total de la composition.

Avantageusement, le tensioactif à faible HLB utilisé dans la composition selon la présente invention comprend, ou est un tensioactif ou un mélange de tensioactifs ayant une valeur HLB allant de 3 à 6, de préférence de 5 à 6.

Avantageusement, le tensioactif à faible HLB est choisi parmi les monoglycérides et les diglycérides d'acides gras à chaîne moyenne, ainsi que les mélanges de ceux-ci. Les mono- et di-glycérides peuvent chacun inclure des mélanges de différents mono- et di-glycérides d'acides gras à chaîne moyenne.

De préférence, le tensioactif à faible HLB est choisi parmi le glycéryl monocaprate, glycéryl dicaprate, glycéryl monocaprylate, glycéryl dicaprylate, glycéryl caprate, glycéryl caprylate, glycéryl monocaprylate/caprate, glycéryl caprylate/caprate, glycéryl dicaprylate/caprate, ou les mélanges de ceux-ci.

Des exemples de ces monoglycérides et diglycérides d'acides gras à chaîne moyenne incluent ceux disponibles sous les noms commerciaux SYMLITE G8 (Symrise) ; CAPMUL (Karlsham Lipid Specialties), par exemple les produits CAPMUL MCM et CAPMUL C8; Cosphaderm® GMCY RSPO/MB (Cosphatec GmbH) ; Glyceryl Caprylate (Cosroma). Généralement, ces tensioactifs peuvent également comprendre une petite quantité en poids d'acides gras à chaîne moyenne libres.

Selon un mode de réalisation préféré, le tensioactif à faible HLB est le glycéryl caprylate.

Selon un mode de réalisation préféré, le rapport massique triglycérides d'acide gras à chaîne moyenne/ tensioactif à faible HLB est inférieur à 1, de préférence inférieur à 0,8, de préférence encore inférieur à 0,7, et de mieux entre 0,40 et 0,65.

Avantageusement, la composition sous forme de microémulsion huile-dans-eau selon l'invention comprend de 5 à 25% en poids, de préférence de 7 à 20% en poids, de préférence encore de 12 à 15% du tensioactif à faible HLB par rapport au poids total de la composition.

Avantageusement, le tensioactif à haut HLB utilisé dans la composition selon l'invention comprend ou est un tensioactif ou un mélange de tensioactifs ayant une valeur HLB allant de 13 à 20, de préférence de 14 à 18.

Avantageusement, le tensioactif à haut HLB comprend un ou plusieurs esters de sorbitane polyéthoxylés, également connus sous le nom de polysorbates. Parmi ces esters esters de sorbitane polyéthoxylés on peut notamment citer le polysorbate 20: monolaurate de sorbitan polyéthoxylé ; le polysorbate 40 : monopalmitate de sorbitan polyéthoxylé, le polysorbate 60 : monostéarate de sorbitan polyéthoxylé ; et le polysorbate 80 : monooléate de sorbitan polyéthoxylé.

Selon un mode de réalisation préféré, le tensioactif à faible HLB est le polysorbate 60 ou le polysorbate 80 ou un mélange des deux, de préférence, le polysorbate 80.

Avantageusement, le rapport massique tensioactif à haut HLB/ (triglycéride d'acide gras à chaîne moyenne + du tensioactif à faible HLB) dans la composition selon l'invention est supérieur ou égal à 0,85.

De préférence, le rapport massique tensioactif à haut HLB/ (triglycéride d'acide gras à chaîne moyenne + du tensioactif à faible HLB) va de 0,9 à 5, de préférence de 0,9 à 2, de préférence encore de 0,95 à 1,5.

Avantageusement, la quantité en poids de tensioactif à haut HLB dans la composition selon l'invention est supérieure à celle du tensioactif à faible HLB, laquelle est elle-même supérieure à celle du triglycéride d'acide gras à chaîne moyenne.

Selon un mode de réalisation préféré, le rapport massique tensioactif à haut HLB/ triglycéride d'acide gras à chaîne moyenne est supérieur ou égal à 2, de préférence supérieur ou égal à 2,5, de préférence entre 2,5 et 5.

Selon un mode de réalisation particulier, le rapport massique tensioactif à haut HLB/ triglycéride d'acide gras à chaîne moyenne est supérieur ou égal à 2 et le rapport massique tensioactif à haut HLB/ (triglycéride d'acide gras à chaîne moyenne + du tensioactif à faible HLB) dans la composition selon l'invention est supérieur ou égal à 0,85.

Avantageusement, le rapport massique du tensioactif à haut HLB au tensioactif à faible HLB dans la composition selon l'invention est entre 1,5 et 3, de préférence entre 1,5 et 2, de préférence encore entre 1,6 et 1,8.

Avantageusement, la composition sous forme de microémulsion huile-dans-eau selon l'invention comprend de 5 à 30% en poids, de préférence de 15 à 25% en poids, de préférence encore de 18 à 24% en poids, et de préférence encore de 20 à 24% en poids de le tensioactif à haut HLB par rapport au poids total de la composition.

La composition selon l'invention comprend également un diol qui peut être choisi parmi le 1,3-propanediol et le propylène glycol (également connu sous le nom de propane-1,2-diol, 1,2-dihydroxypropane ou méthyl glycol). De préférence, le diol est le 1,3-propanediol.

Selon un mode de réalisation préféré, le rapport massique diol/ triglycéride d'acide gras à chaîne moyenne est supérieur ou égale à 0,6, de préférence supérieur ou égal à 0,9, de préférence encore supérieur ou égal à 1, et de préférence encore entre 1 et 2.

Avantageusement, la composition selon l'invention comprend de 3% à 20% en poids, de préférence de 3% à 15% en poids, de préférence encore de 4% à 12% en poids, de préférence encore de 5% à 10% en poids de diol par rapport au poids total de la composition.

Selon un mode de réalisation particulier, la composition selon l'invention comprend de 3% à 20% en poids de diol et le rapport massique tensioactif à haut HLB/ (triglycéride d'acide gras à chaîne moyenne + du tensioactif à faible HLB) dans la composition selon l'invention est supérieur ou égal à 0,85.

L'eau utilisée dans la composition selon l'invention peut être de l'eau distillée, de l'eau déionisée ou de l'eau osmosée. Une eau convenant à l'invention peut être aussi être une eau de source naturelle ou une eau florale.

Avantageusement, la composition selon l'invention comprend au moins 40% en poids, de préférence entre 40% et 80%, de préférence encore entre 42 et 56% en poids d'eau par rapport à la masse totale de la composition.

Selon un mode particulièrement préféré de l'invention, la composition comprend au moins :

- de 7% à 9% en poids de triglycéride d'acide gras à chaîne moyenne ;
- de 12% à 15% en poids de tensioactif à faible HLB ;
- de 20% à 24% en poids de tensioactif à haut HLB ;
- de 5% à 10% en poids de diol ; et
- de 42% à 56% en poids d'eau.

Selon un mode de réalisation, la composition selon l'invention consiste essentiellement en :

- un triglycéride d'acide gras à chaîne moyenne ;
- un tensioactif à faible HLB ;
- un tensioactif à haut HLB ;
- un diol choisi parmi le1,3-propanediol et le propylène glycol; et
- de l'eau

Selon un mode particulièrement préféré de l'invention, la composition consiste essentiellement en:

- de 7% à 9% en poids de triglycéride d'acide gras à chaîne moyenne
- de 12% à 15% en poids de tensioactif à faible HLB;
- de 20% à 24% en poids de tensioactif à haut HLB ;
- de 5% à 10% en poids de diol choisi parmi le1,3-propanediol et le propylène glycol ; et
- de 42% à 56% en poids d'eau.

La composition sous forme de microémulsion de la présente invention est transparente ou translucide, donc non opaque.

Par "microémulsion transparente ou translucide", on entend selon l'invention, une microémulsion dont la matrice laisse passer la lumière sans provoquer de déviation par réfraction ou réflexion, ou en ne provoquant que de faibles déviations des rayons lumineux à l'interface des deux phases lipophile et hydrophile.

Le terme « transparent » désigne la propriété physique permettant à la lumière de traverser un matériau sans être dispersée, suivant la loi de Snell, ce qui permet de voir clairement à travers ce matériau. Le terme « translucide » inclut la transparence, mais permet à la lumière de passer avec une dispersion partielle. Cela crée une apparence floue ou voilée, car la lumière est partiellement dispersée lorsqu'elle traverse le matériau.

La transparence ou la translucidité de la composition selon l'invention peuvent être évaluées à l'œil nu ou mesurées à l'aide d'un turbidimètre.

De préférence, le diamètre des gouttelettes de la phase lipophile dans la microémulsion de la présente invention, est inférieur à 100 nm, de préférence inférieur à 80 nm, de préférence encore inférieur à 50 nm, de mieux inférieure à 40 nm.

La taille des gouttelettes de la phase lipophile peut être déterminée en tant que taille moyenne Z ou taille moyenne pondérée par l'intensité, à l'aide de techniques bien connues par la personne du métier, en particulier par diffusion dynamique de la lumière (DLS).

La composition selon l'invention présente une excellente stabilité au cours du temps et notamment pendant le transport et le stockage.

Par « stabilité » ou « composition stable », on entend désigner, au sens de la présente invention, l'absence de crémage (remontée vers la surface) ou de sédimentation (précipitation vers le fond) des gouttes de la phase dispersée dans la phase continue, l'absence de coalescence (fusion des gouttelettes), l'absence d'opacification de la phase continue et/ou l'absence de fuite de matières de la phase dispersée vers la phase continue, ou inversement.

En particulier, la composition selon l'invention présente l'avantage de rester transparente ou translucide à basse température et à température ambiante, sans séparation de phases, trouble ou précipitation, même sur de très longues périodes.

Avantageusement, la composition selon l'invention peut être stockée et/ou transportée sous une forme stable à diverses températures, en particulier à basse température, par exemple à de 0 à 15°C, à température ambiante, par exemple de 15°C à 37°C et à température élevée, par exemple de 40 à 60°C.

Avantageusement, la composition selon l'invention peut être stockée et/ou transportée sous une forme stable pendant au moins 3 mois, de préférence au moins 6 mois, de préférence encore 12 mois, de préférence encore 24 mois et encore mieux au moins 36 mois ou mieux à 72 mois.

Selon un mode de réalisation, la composition selon l'invention peut être stockée et/ou transportée sous une forme stable pendant au moins 3 mois, de préférence au moins 6 mois, de préférence encore 12 mois, de préférence encore 24 mois et encore mieux au moins 36 mois à une température allant de 0 à 15°C.

Selon un autre mode de réalisation, la composition selon l'invention peut être stockée et/ou transportée sous une forme stable pendant au moins 3 mois, de préférence au moins 6 mois, de préférence encore 12 mois, de préférence encore 24 mois et encore mieux au moins 36 mois à une température de 15 à 37°C.

Selon un autre mode de réalisation, la composition selon l'invention peut être stockée et/ou transportée sous une forme stable pendant au moins 3 mois, de préférence au moins 6 mois, de préférence encore 12 mois, de préférence encore 24 mois et encore mieux au moins 36 mois à une température de 40 à 60°C.

De manière avantageuse, la composition selon l'invention comprend en outre au moins un agent cosmétiquement et/ou dermatologiquement acceptable.

Par "cosmétiquement et/ou dermatologiquement acceptable", on entend désigner un composé et/ou milieu qui est généralement sûr, non toxique et ni biologiquement ni autrement non souhaitable, convenant particulièrement à une application cosmétique et/ou dermatologique notamment par voie topique.

En particulier, l'agent cosmétiquement et/ou dermatologiquement acceptable peut être choisi parmi les épaississants, conservateurs, parfums, huiles essentielles, colorants, agents gélifiants, pigments, céramiques, humectants, stabilisateurs, émollients, et modificateurs de pH, de force osmotique et d'indice de réfraction et/ou d'autres additifs cosmétiques.

La composition selon l'invention peut également comprendre au moins un actif biologique, notamment choisi parmi les agents hydratants, cicatrisants, anti-radicaux libres, dépigmentants, mélanorégulateurs, filtres UV, accélérateurs de bronzage, desquamants, antioxydants, dermodécontractants, anti-transpirants, apaisants, anti-âge, amincissants, anti-acnéiques, anti-séborrhéiques, antipelliculaires, anti-vieillissement, anti-rides, kératolytiques, anti-inflammatoires, rafraîchissants, anti-chute des cheveux, antibactériens, antifongiques, antiperspirants, ainsi que leurs mélanges.

Selon un mode de réalisation préféré, la composition selon l'invention comprend un actif biologique choisi parmi les dépigmentants, les anti-acnéiques, les anti-vieillissements, les anti-rides et leurs mélanges.

Selon un mode de réalisation, la composition selon l'invention comprend en outre un actif dépigmentant choisi parmi les dérivés de l'hydroquinone tels que l'arbutine, la désoxyarbutine, et l'alpha-arbutine ;l'acide ascorbique, l'acide tranexamique et ses dérivés, l'acide glycolique, l'acide kojique, l'acide azélaïque et ses dérivés, l'acide rétinoïque, la niacinamide, le glutathione, les polyphénols tels que le resvératrol et les isoflavones comme la glabridine (extrait de racine de réglisse) , les dérivé du résorcinol tel que le phényléthyl résorcinol, ainsi que mélanges de ces actifs.

De préférence, l'actif dépigmentant est choisi parmi le mésylate de tranexamate de cétyle, le glutathione, le phenylethyl resorcinol et leurs mélanges.

Selon un autre mode de réalisation préféré, la composition selon l'invention comprend en outre un actif antivieillissement cutané ou anti-rides choisi parmi la vitamine A et ses dérivés (le rétinol, la tretinoïne (acide rétinoïque)), le matrixyl (palmitoyl pentapeptide-4), l'acide hyaluronique, la vitamine C (acide ascorbique), la vitamine E (tocophérol), les AHA (acides alpha-hydroxylés) tels que l'acide glycolique et les BHA (acides bêta-hydroxylés), la coenzyme Q10, la vitamine B3 (niacinamide), l'extrait de thé vert, l'acide alpha-lipoïque, ainsi que les dérivés de ceux-ci et leurs mélanges. De préférence, l'actif antivieillissement cutané est choisi parmi les ester d'acide rétinoïque, l'acétyle hexapeptide-8, l'hydroxyprolisilane et leurs mélanges.

Selon un autre mode de réalisation préféré, la composition selon l'invention comprend en outre un actif anti-acné choisi parmi l'acide salicylique, l'acide shikimique, le peroxyde de benzoyle, les rétinoïdes, la niacinamide, l'acide azélaïque et ses dérivés, l'acide glycolique, le soufre, l'extrait de thé vert, la clindamycine, l'eau de rose, le zinc, les PHA (acides poly-hydroxylés) ainsi que les dérivés de ceux-ci et leurs mélanges. De préférence, l'actif anti-acné est choisi parmi l'acide shikimique, l'acide succinique, l'acide capryloyl salicylique et leurs mélanges.

De préférence, la composition selon l'invention comprend de 0,01 à 15% en poids, de préférence de 0,5 à 10% en poids de préférence encore de 1 à 5% en poids d'un ou de plusieurs actifs biologiques tels que décrits ci-dessus.

Les agents cosmétiquement et/ou dermatologiquement acceptable ou encore les actifs biologiques peuvent être ajoutés dans la composition sous fourme libre ou encapsulée.

Bien entendu, la personne du métier saura choisir les éventuels composé(s) additionnel(s) et/ou leur quantité de telle manière que les propriétés avantageuses et la stabilité de la composition selon l'invention ne soient pas ou substantiellement pas altérées.

La composition selon l'invention peut également comporter un ou plusieurs principes actifs pharmaceutiques.

La composition sous forme de microémulsion selon l'invention peut être préparée en mélangeant les différents composés tels que définis ci-dessus par les techniques connues de la personne du métier. L'ordre d'addition des composés, la vitesse ou encore le type de mélange ne sont pas critiques pour la préparation de la composition selon l'invention.

De préférence, la microémulsion huile-dans-eau selon la présente invention se forme spontanément lorsqu'on met en présence les ingrédients tels que définis ci-dessus. On sait que la microémulsion est formée lorsque le mélange devient transparent ou translucide. De préférence, le procédé de préparation de la composition selon l'invention ne comprend pas d'étape de chauffage.

La composition peut être préparée, par exemple, par addition progressive du tensioactif à haut HLB à un mélange comprenant le triglycéride d'acide gras à chaine moyenne, le 1,3-propanediol, le tensioactif à faible HLB et éventuellement les additifs ou actifs biologiques, tout en agitant le milieu réactionnel.

Utilisations de la composition selon l'invention

L'invention a également pour objet l'utilisation d'une composition sous forme de microémulsion huile-dans-eau telle que décrite en détail ci-dessus comme produit de soin et/ou d'hygiène et/ou de maquillage des matières kératiniques, notamment de la peau et des cheveux.

Par « matières kératiniques », on entend au sens de l'invention la peau, notamment du corps ou du visage, les cheveux, les ongles, le cuir chevelu et les poils.

La composition selon l'invention peut être ainsi utilisée comme base pour des produits sous la forme d'une lotion liquide, d'un sérum, d'une brume, d'une eau micellaire. La composition selon l'invention peut également servir pour imbiber des lingettes de démaquillage.

La composition selon l'invention peut être utilisée dans toutes les applications externes cutanées (topiques) et/ou internes (orales, injectables). De préférence, la composition est utilisée pour un usage topique externe.

L'invention a également pour objet l'utilisation cosmétique non thérapeutique d'une composition telle que décrite ci-dessus pour améliorer la fermeté et/ou l'élasticité de la peau et/ou améliorer le microrelief cutané et/ou améliorer l'éclat de la peau.

L'invention a également pour objet l'utilisation cosmétique non thérapeutique d'une composition telle que décrite ci-dessus pour prévenir et/ou traiter les signes du vieillissement cutané, de préférence pour réduire et/ou éliminer et/ou atténuer les rides et les ridules.

L'invention a également pour objet l'utilisation cosmétique non thérapeutique d'une composition telle que décrite ci-dessus pour prévenir et/ou réduire et/ou éliminer l'acné, en particulier la bactérie Cutibacterium acnes, et/ou pour favoriser l'élimination des effets cosmétiques de l'acné en particulier les taches d'acné.

L'invention a également pour objet l'utilisation cosmétique non thérapeutique d'une composition telle que décrite ci-dessus pour prévenir et/ou réduire et/ou atténuer et/ou éliminer les taches de pigmentation, et/ou réduire leur taille, et/ou unifier et/ou illuminer et/ou éclaircir le teint.

L'invention a encore pour objet un procédé de traitement cosmétique non thérapeutique consistant à appliquer la composition selon l'invention sur les matières kératiniques, notamment sur la peau.

**Exemples** :

L'invention est illustrée par les exemples suivants donnés à titre non limitatif.

Dans les exemples, les parties et pourcentages sont exprimés en poids sauf indication contraire.

1- Matériaux et méthodes :

Préparation des microémulsions :

Les compositions selon l'invention ont été préparées en réalisant un premier mélange d'eau et de 1,3 propanediol (M1) et un second mélange comprenant le glyceryl caprylate, le caprylic/capric triglycéride et le polysorbate 80 (M2). Les deux solutions, M1 et M2, ont été agitées jusqu'à homogénéisation, puis le mélange M2 a été ajouté lentement au mélange M1 tout en continuant à agiter avec un agitateur magnétique. La liste des ingrédients utilisés est présentée dans le Tableau 1.

Tableau 1 : Ingrédients utilisés et leurs noms INCI

| Ingrédient | Nom INCI | Nom commercial et fabricant |
|---|---|---|
| Eau osmosée | WATER | Dermosciences |
| Tensioactif à haut HLB | POLYSORBATE 80 | EUMULGIN SMO 20, BASF |
| Triglycéride d'acide gras à chaîne moyenne | CAPRYLIC/CAPRIC TRIGLYCERIDE | MYRITOL 318, Ami Ingredients |
| Diol | 1,3-PROPANEDIOL | TILAMAR PDO WITH NOOVISTA, les Ingredients |
| Tensioactif à faible HLB | GLYCERYL CAPRYLATE | SYMLITE G8, Symrise |

Protocole de mesure de la stabilité des microémulsions :

Des tests de stabilité ont été réalisés sur une période de 6 mois dans des conditions accélérées à 5°C $\pm$ 3°C, 25°C $\pm$ 5°C et 45°C $\pm$ 2°C. L'objectif de ce test de stabilité accélérée est de déterminer le temps de dégradation de la microémulsion. Environ 15 ml de chaque composition préparée sont transvasé dans 3 pots en verres non ambrés de 30 ml. Chaque pot a été entreposé à la fois dans un bac où la température de la pièce est réglée à 25°C $\pm$ 5°C et dans des étuves à 5°C $\pm$ 3°C (étuve « Aqualytic Refrigerator TC 135 S ») et à 45°C $\pm$ 2°C (étuve « Memmert Heating Oven UF55 »).
L'aspect, la couleur et l'odeur sont évalués aux échéances suivantes : 7 jours, 14 jours ,1 mois, 1 mois et demi, 3 mois et 6 mois. A chaque échéance, les compositions ont été examinées à l'œil nu en sortie d'étuve et une fois revenue à température ambiante de la pièce (après quelques heures de repos).
Si, par exemple, la composition conservée à 45°C reste stable pendant trois mois, on peut lui accorder une durée de stabilité jusqu'à 36 mois. Si la composition conservée à 45°C reste stable pendant six mois, on peut lui accorder une durée de stabilité jusqu'à 6 ans.

2- Résultats

Les différentes compositions testées ainsi que les résultats de stabilité sont présentés dans le Tableau 2. Les compositions E15, E31, E32 et E33 sont selon l'invention. La composition E13 est comparative.
Les émulsions sont considérées comme stables au stockage si elles ne montrent pas des signes d'instabilité (changement de couleurs, d'odeur et/ou d'aspect transparent) jusqu'à 3 mois de conservation à température à 45°C. Les résultats montrent que la composition E13, sans diol, perd sa stabilité après 2 mois à 45°C. En revanche, les compositions E15, E31, E32 et E33, selon l'invention, restent stables pendant 3 mois à 45°C, ce qui correspond à une stabilité de 3 ans à température ambiante.
D'autres compositions E17, E18, E19, E20 et E30 ont également été évaluées en faisant varier le rapport massique entre le tensioactif à haut HLB et le mélange (triglycéride d'acide gras à chaîne moyenne + tensioactif à faible HLB). Ces compositions présentant un rapport inférieur à 0,85, montrent des signes d'instabilité plus précoces, même à température ambiante.

Tableau 2 : Stabilité des compositions testées

|  | E13 | E15 | E31 | E32 | E33 |
|---|---|---|---|---|---|
| Caprylic/Capric Triglyceride (a) (%) | 7,5 | 7,5 | 7,5 | 7,5 | 8,5 |
| Glyceryl Caprylate (b) (%) | 12,5 | 12,5 | 12,5 | 12,5 | 14 |
| Polysorbate 80 (c) (%) | 20 | 20 | 20 | 20 | 22,5 |
| 1,3-propanediol (d) (%) | 0 | 5 | 7,5 | 10 | 10 |
| Eau (e) (%) | 60 | 55 | 52,5 | 50 | 45 |
| Rapport (c) / ((a) + (b)) | 1 | 1 | 1 | 1 | 1 |
| Stabilité | 2 mois à 45°C | 3 mois à 45°C | 6 mois à 45°C | 6 mois à 45°C | 6 mois à 45°C |

Tableau 2 : Stabilité des compositions testées (suite)

| | E17 | E18 | E19 | E20 | E30 |
|---|---|---|---|---|---|
| Caprylic/Capric Triglyceride (a) (%) | 7,5 | 7,5 | 8,5 | 8,5 | 12,5 |
| Glyceryl Caprylate (b) (%) | 12,5 | 12,5 | 14 | 14 | 12,5 |
| Polysorbate 80 (c) (%) | 12,5 | 15 | 15 | 12,5 | 20 |
| 1,3-propanediol (d) (%) | 2,5 | 5 | 5 | 5 | 5 |
| Eau (e) (%) | 65 | 60 | 57.5 | 60 | 50 |
| Rapport (c) / ((a) + (b)) | 0,63 | 0,75 | 0,67 | 0,56 | 0,80 |
| Stabilité | 4°C : trouble | 2mois à 45°C : trouble | J14 à 45°C: trouble | 25°C : trouble | 4°C : trouble |

**Revendications**

1. Composition sous forme de microémulsion huile-dans-eau comprenant au moins :

   a) un triglycéride d'acide gras à chaîne moyenne ayant de 6 à 12 atomes de carbone;
   b) un tensioactif à faible HLB ayant un HLB allant de 3 à 6 ;
   c) un tensioactif à haut HLB ayant un HLB allant de 13 à 20;
   d) un diol choisi parmi le 1,3-propanediol et le propylène glycol ;
   e) de l'eau,

   dans laquelle le rapport massique du tensioactif à haut HLB au mélange (triglycéride d'acide gras à chaîne moyenne ayant de 6 à 12 atomes de carbone + tensioactif à faible HLB) est supérieur ou égal à 0,85.

2. Composition selon la revendication 1, dans laquelle le tensioactif à faible HLB a un HLB allant de 5 à 6 et/ou le tensioactif à haut HLB a un HLB allant de 14 à 18.

3. Composition selon la revendication 1 ou la revendication 2, dans laquelle le rapport massique du tensioactif à haut HLB au mélange (triglycéride d'acide gras à chaîne moyenne + du tensioactif à faible HLB) va de 0,90 à 5, de préférence de 0,90 à 2.

4. Composition selon l'une quelconque des revendications précédentes, dans laquelle le rapport massique triglycérides d'acide gras à chaîne moyenne/ tensioactif à faible HLB est inférieur à 1, de préférence inférieur à 0,8, de préférence encore inférieur à 0,7, et de mieux entre 0,40 et 0,65.

5. Composition selon l'une quelconque des revendications précédentes, dans laquelle le rapport massique du tensioactif à haut HLB/tensioactif à faible HLB est entre 1,5 et 3, de préférence entre 1,5 et 2, de préférence encore entre 1,6 et 1,8.

6. Composition selon l'une quelconque des revendications précédentes, dans laquelle le rapport massique diol/ triglycéride d'acide gras à chaîne moyenne est supérieur ou égale à 0,6, de préférence supérieur ou égal à 0,9, de préférence encore supérieur ou égal à 1, et de préférence encore entre 1 et 2.

7. Composition selon l'une quelconque des revendications précédentes, comprenant de 3% à 20% en poids, de préférence de 3% à 15% en poids, de préférence encore de 4% à 12% en poids, de préférence encore de 5 à 10% en poids de diol par rapport au poids total de la composition.

8. Composition selon l'une quelconque des revendications précédentes, dans laquelle le diol est le 1,3-propanediol.

9. Composition selon l'une quelconque des revendications précédentes, dans laquelle le triglycéride d'acide gras à chaîne moyenne ayant de 6 à 12 atomes de carbone est le triglycéride caprylique/caprique.

10. Composition selon l'une quelconque des revendications précédentes, dans laquelle le tensioactif à faible HLB comprend un monoglycéride et/ou un diglycéride d'acide gras à chaîne moyenne choisi parmi le glycéryl monocaprate, glycéryl dicaprate, glycéryl monocaprylate, glycéryl dicaprylate, glycéryl caprate, glycéryl caprylate, glycéryl monocaprylate/caprate, glycéryl caprylate/caprate, glycéryl dicaprylate/caprate, de préférence le glycéryl caprylate.

11. Composition selon l'une quelconque des revendications précédentes, dans laquelle le tensioactif à haut HLB comprend un ester de sorbitane éthoxylé.

12. Composition selon la revendication 11 où le tensioactif à haut HLB est le polysorbate 80.

13. Composition selon l'une quelconque des revendications précédentes, comprenant, par rapport au poids total de la composition :

   a) de 7% à 9% en poids de triglycéride ;
   b) de 12% à 15% en poids de tensioactif à faible HLB ;
   c) de 20% à 24% en poids de tensioactif à haut HLB ;
   d) de 5% à 10% en poids de diol ; et
   e) de 42% à 56% en poids d'eau.

14. Composition selon l'une quelconque des revendications précédentes, comprenant en outre au moins un agent cosmétiquement et/ou dermatologiquement acceptable et/ou au moins un actif biologique.

15. Utilisation d'une composition selon l'une quelconque des revendications précédentes comme produit de maquillage, d'hygiène, de nettoyage et/ou de soin des matières kératiniques, de préférence de la peau.

Europäisches
Patentamt

European
Patent Office

Office européen
des brevets

# RAPPORT DE RECHERCHE EUROPEENNE

Numéro de la demande

EP 25 21 2939

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 2011/159104 A1 (TESLENKO ALEXANDER [DE]) 30 juin 2011 (2011-06-30) <br> * Composition 5; <br> exemples 1,7; tableau 1 * <br> * revendication 13 * <br> ----- | 1-15 | INV. <br> A61K8/06 <br> A61K8/34 <br> A61K8/37 <br> A61K8/49 <br> A61Q1/00 |
| Y | SHAH ANKITA ET AL: "Effect of different polysorbates on development of self-microemulsifying drug delivery systems using medium chain lipids", JOURNAL DRUG DEVELOPMENT AND INDUSTRIAL PHARMACY , vol. 44, no. 2 23 octobre 2017 (2017-10-23), pages 215-223, XP055953710, US ISSN: 0363-9045, DOI: 10.1080/03639045.2017.1386202 Extrait de l'Internet: URL:https://www.tandfonline.com/doi/pdf/10 .1080/03639045.2017.1386202?needAccess=tru e [extrait le 2017-10-23] * le document en entier * ----- | 1-15 | A61Q19/00 <br> A61Q19/10 |
| Y | US 2018/036233 A1 (SHABAIK YUMNA [US] ET AL) 8 février 2018 (2018-02-08) * revendications 1,4,9,10,11,13,37,39 * * alinéa [0067] * * figures 2-13 * * exemple 8 * ----- | 1-15 | DOMAINES TECHNIQUES RECHERCHES (IPC) <br><br> A61K <br> A61Q |
| Y | US 2009/221625 A1 (HIRSCH STEFAN [DE] ET AL) 3 septembre 2009 (2009-09-03) * exemple 3 * ----- | 1-15 | |

Le présent rapport a été établi pour toutes les revendications

| Lieu de la recherche | Date d'achèvement de la recherche | Examinateur |
|---|---|---|
| Munich | 11 mars 2026 | Grenouillat, N |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
............................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 25 21 2939

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-03-2026

| Document brevet cité au rapport de recherche | Date de publication | Membre(s) de la famille de brevet(s) | Date de publication |
|---|---|---|---|
| US 2011159104 A1 | 30-06-2011 | DE 102008034944 A1 | 28-01-2010 |
| | | EP 2320754 A2 | 18-05-2011 |
| | | US 2011159104 A1 | 30-06-2011 |
| | | WO 2010012408 A2 | 04-02-2010 |
| US 2018036233 A1 | 08-02-2018 | AU 2016226224 A1 | 07-09-2017 |
| | | CA 2976952 A1 | 09-09-2016 |
| | | CN 107257680 A | 17-10-2017 |
| | | CN 116270460 A | 23-06-2023 |
| | | EP 3265056 A1 | 10-01-2018 |
| | | HK 1245135 A1 | 24-08-2018 |
| | | JP 7105338 B2 | 22-07-2022 |
| | | JP 7187150 B2 | 12-12-2022 |
| | | JP 2018507239 A | 15-03-2018 |
| | | JP 2021107402 A | 29-07-2021 |
| | | JP 2022145687 A | 04-10-2022 |
| | | KR 20170120161 A | 30-10-2017 |
| | | KR 20240005193 A | 11-01-2024 |
| | | RU 2017129930 A | 05-04-2019 |
| | | US 2018036233 A1 | 08-02-2018 |
| | | US 2022218599 A1 | 14-07-2022 |
| | | US 2024307299 A1 | 19-09-2024 |
| | | WO 2016141098 A1 | 09-09-2016 |
| US 2009221625 A1 | 03-09-2009 | BR PI0611155 A2 | 17-08-2010 |
| | | CA 2605768 A1 | 09-11-2006 |
| | | CN 101166509 A | 23-04-2008 |
| | | EP 1874260 A1 | 09-01-2008 |
| | | US 2009221625 A1 | 03-09-2009 |
| | | WO 2006117132 A1 | 09-11-2006 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- US 2011159104 A

**Littérature non-brevet citée dans la description**

- **A. SHAH et al.** *Drug Development and Industrial Pharmacy*, 2017, vol. 44, 215-223

- **GRIFFIN** ; **WILLIAM C**. Calculation of HLB Values of Non-Ionic Surfactants. *Journal of the Society of Cosmetic Chemists*, 1954, vol. 5 (4), 249-56